(19) Europäisches Patentamt
European Patent Office
Office européen des brevets

(11) Veröffentlichungsnummer: **0 265 685 B1**

(12) # EUROPÄISCHE PATENTSCHRIFT

(45) Veröffentlichungstag der Patentschrift: **29.07.92**

(51) Int. Cl.[5]: **A61K 37/64**, //(A61K37/64, 31:44,31:54)

(21) Anmeldenummer: **87114164.4**

(22) Anmeldetag: **29.09.87**

Die Akte enthält technische Angaben, die nach dem Eingang der Anmeldung eingereicht wurden und die nicht in dieser Patentschrift enthalten sind.

(54) **Kombination von Angiotensin- Converting-Enzyme-Hemmern mit Calciumantagonisten sowie deren Verwendung in Arzneimitteln.**

(30) Priorität: **02.10.86 DE 3633496**

(43) Veröffentlichungstag der Anmeldung:
**04.05.88 Patentblatt 88/18**

(45) Bekanntmachung des Hinweises auf die Patenterteilung:
**29.07.92 Patentblatt 92/31**

(84) Benannte Vertragsstaaten:
**AT BE CH DE ES FR GB GR IT LI LU NL SE**

(56) Entgegenhaltungen:
**EP-A- 0 180 785**
**EP-A- 0 257 485**

**BIOLOGICAL ABSTRACTS/RRM, Nr. 30061407; O.M. LUOUE: "Combination of antihypertensive drugs", & REV PHARMACOL CLIN EXP 1985, Band 2, Nr. 3, Seiten 197-198**

(73) Patentinhaber: **HOECHST AKTIENGESELLSCHAFT**
**Postfach 80 03 20**
**W-6230 Frankfurt am Main 80(DE)**

(72) Erfinder: **Becker, Reinhard, Dr.**
**Adelheidstrasse 101**
**W-6200 Wiesbaden(DE)**
Erfinder: **Henning, Rainer, Dr.**
**Rotenhofstrasse 31**
**W-6234 Hattersheim am Main(DE)**
Erfinder: **Rüger, Wolfgang, Dr.**
**Parkstrasse 10**
**W-6233 Kelkheim Taunus(DE)**
Erfinder: **Teetz, Wolfgang, Dr.**
**An der Tann 20**
**W-6238 Hofheim am Taunus(DE)**
Erfinder: **Urbach, Hansjörg, Dr.**
**Le Lavandoustrasse 41**
**W-6242 Kronberg/Taunus(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

DIALOG, Nr. 06119563, MEDLINE 87093563; A. SIMON: "Hypertension artérielle essentielle: stratégie d'emploi des médicaments antihypertenseurs", & REV. MED. INTERNE (FRANCE), September 1986, 7(4), Seiten 433-440

CHEMICAL ABSTRACTS, Band 102, Nr. 1, 7. Januar 1985, Seite 35, Nr. 372x, Columbus, Ohio, US; M.E. VINCENT et al.: "Hemodynamic and humoral responses to enalapril and nifedipine in the rat", & CLIN. EXP. HYPERTENS., PART A, 1984, A6(8), 1485-1497

DIALOG, Nr. 5828999, EMBASE Nr. 85074509; R.M.L. BROUWER et al.: "Antihypertensive treatment using calcium antagonists in combination with captopril rather than diuretics", & J. CARDIOVASC. PHARMACOL. (USA), 1985, 7/SUPPL 1 (s88-s91).

## Beschreibung

Die vorliegende Erfindung betrifft eine Kombination von Angiotensin-Converting-Enzyme-Hemmern (ACE-Hemmer) mit Calciumantagonisten sowie ihre Verwendung in Arzneimitteln, insbesondere in blutdrucksenkenden Arzneimitteln. ACE-Hemmer sind Verbindungen, die die Umwandlung von Angiotensin I in das pressorisch wirksame Angiotensin II verhindern. Solche Verbindungen sind beispielsweise in den folgenden Patentanmeldungen oder Patenten beschrieben:
US-PS 4 350 633, US-PS 4 344 949, US-PS 4 294 832, US-PS 4 350 704, EP-A 50 800, EP-A 31 741, EP-A 51 020, EP-A 49 658, EP-A 49 605, EP-A 29 488, EP-A 46 953, EP-A 52 870.

Sie sind weiter Gegenstand der deutschen Patentanmeldungen P 32 26 768.1, P 31 51 690.4, P 32 10 496.0, P 32 11 397.8, P 32 11 676.4, P 32 27 055.0, P 32 42 151.6, P 32 46 503.3, P 32 46 757.5.

Ihre blutdrucksenkende Wirkung ist gut dokumentiert. Calciumantagonisten sind solche Verbindungen, die den Einstrom von Calcium-Ionen in Zellen, insbesondere glatte Muskelzellen beeinflussen. Solche Verbindungen sowie ihre blutdrucksenkende Wirkung sind in einer Vielzahl von Publikation und Patentanmeldungen niedergelegt.

Da beide Substanzgruppen in verschiedene Blutdruckregulationssysteme eingreifen, wird bei einer kombinierten Anwendung der Effekt des einen Kombinationspartners durch den jeweiligen anderen Partner gesteigert. Dies führt bei einer kombinierten Anwendung zu einer Verringerung der Dosis der jeweiligen Kombinationspartner, verglichen mit der Einzelanwendung. Dadurch kann das Auftreten der für die beiden Substanzklassen bekannten Nebenwirkungen verringert bzw. vermieden werden.
Kombinationen von Enalapril mit Calciumantagonisten aus der Strukturklasse der Dihydropyridine sind in der EP-A-180785 beschrieben, die Kombination des ACE-Inhibitors Enalapril mit dem Nitrophenylderivat Nifedipin wird von M.E. Vincent in Chemical Abstracts, 102, Januar 1985, Seite 35, Nr. 372 x offenbart.

Ferner werden in EP-A 0 257 485 Kombinationen von ACE-Inhibitoren mit einem inotrop wirkenden Dihydropyridin-Derivat beschrieben.

Die vorliegende Erfindung betrifft die Kombination von ACE-Hemmern, die ein bicyclisches oder tricyclisches System enthalten, mit speziellen Calciumantagonisten, wobei diese Kombination zu einer besonders starken blutdrucksenkenden Wirkung führt.

Als ACE-Hemmer kommen die folgenden Verbindungen der Formel I oder deren physiologisch verträglichen Salze in Betracht:

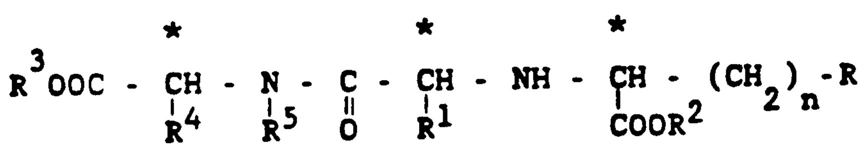

(I)

in welcher

n = 1 oder 2 ist,

R = $(C_1-C_6)$-Alkyl, $(C_2-C_6)$-Alkenyl, $(C_3-C_9)$-Cycloalkyl, Amino-$(C_1-C_4)$-alkyl, $(C_2-C_5)$-Acylamino-$(C_1-C_4)$-alkyl, $(C_7-C_{13})$-Aroylamino-$(C_1-C_4)$-alkyl, $(C_1-C_4)$-Alkoxycarbonylamino-$(C_1-C_4)$-alkyl, $(C_6-C_{12})$-Aryl-$(C_1-C_4)$-alkoxycarbonylamino-$(C_1-C_4)$-alkyl, $(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino und/oder Methylendioxy mono-, di- oder trisubstituiert sein kann, oder 3-Indolyl, insbesondere Methyl, Ethyl, Cyclohexyl, tert. Butoxycarbonylamino$(C_1-C_4)$-alkyl, Benzoyloxycarbonylamino-$(C_1-C_4)$-alkyl oder Phenyl, das durch Phenyl, $(C_1-C_2)$-Alkyl, ($C_1$ oder $C_2$)-Alkoxy, Hydroxy, Fluor, Chlor, Brom, Amino, $(C_1-C_4)$-Alkylamino, Di-$(C_1-C_4)$alkylamino, Nitro und/ oder Methylendioxy mono- oder disubstituiert oder im Falle von Methoxy, trisubstituiert sein kann, bedeutet.

$R^1$ wasserstoff oder $(C_1-C_6)$-Alkyl, das gegebenenfalls durch Amino, $(C_1-C_6)$-Acylamino oder Benzoylamino substituiert sein kann, $(C_2-C_6)$-Alkenyl, $(C_3-C_9)$-Cycloalkyl, $(C_5-C_9)$-Cycloalkenyl, $(C_3-C_7)$-Cycloalkyl-$(C_1-C_4)$-alkyl, $(C_6-C_{12})$-Aryl oder teilhydriertes Aryl, das jeweils durch $(C_1-C_4)$-Alkyl, ($C_1$ oder $C_2$)-Alkoxy oder Halogen substituiert sein kann, $(C_6-C_{12})$-Aryl-($C_1$ bis $C_4$)-alkyl oder $(C_7-C_{13})$-Aroyl-$(C_1-C_2)$-alkyl, die beide wie vorstehend definiert im Arylrest substituiert sein können, ein mono- bzw. bicyclischer Heterocyclen-Rest mit 5 bis 7 bzw. 8 bis 10 Ringatome, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten $\alpha$-Aminosäure, insbesondere aber

Wasserstoff, ($C_1$-$C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, ($C_1$-$C_6$)-Alkyl, ($C_2$-$C_6$)-Alkenyl oder ($C_6$-$C_{12}$)-Aryl-($C_1$-$C_4$)-alkyl, insbesondere aber Wasserstoff, ($C_1$-$C_4$)-Alkyl oder Benzyl bedeuten und

$R^4$ und $R^5$ zusammen mit den diese tragenden Atomen ein Ringsystem aus der Reihe Tetrahydroisochinolin; Decahydroisochinolin; Octahydroindol; Octahydrocyclopenta[b]pyrrol; 2-Azaspiro-[4.5]decan; 2-Azaspiro[4.4]nonan; Spiro[(bicyclo[2.2.1]heptan)-2,3'-pyrrolidin];Spiro[-(bicyclo[2.2.2]-octan)-2,3'-pyrrolidin]; 2-Azatricyclo[4,3,o,$1^{6,9}$]decan; Decahydrocyclohepta-[b]pyrrol; Octahydroisoindol; Octahydrocyclopenta[c]pyrrol; 2,3,3a,4,5,7a-Hexahydroindol; 2-Azabicyclo[3.1.0]-hexan und Hexahydrocyclopenta[b]pyrrol, die alle gegebenenfalls substituiert sein können, bilden.

Bei den Verbindungen, die mehrere chirale Atome besitzen, kommen alle möglichen Diastereomere als Racemate oder Enantiomere, oder Gemische verschiedener Diastereomere in Betracht.

Die in Betracht kommenden cyclischen Aminosäureester weisen die folgenden Strukturformeln auf.

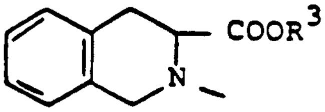
A

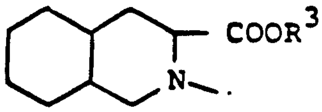
B

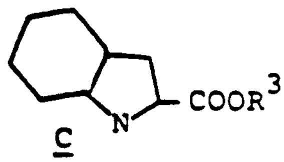
C

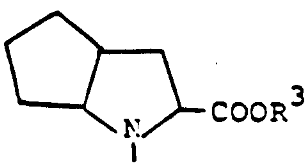
D

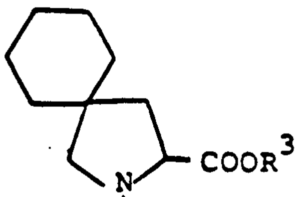
E

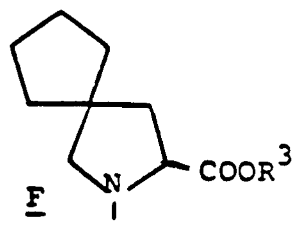
F

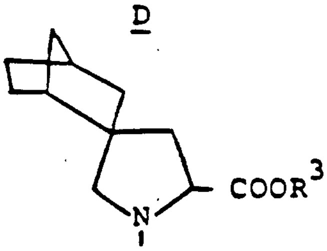
G

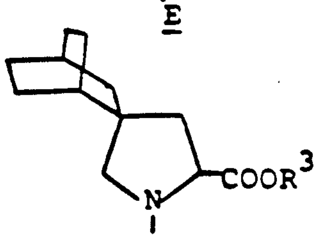
H

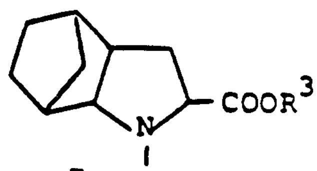
I

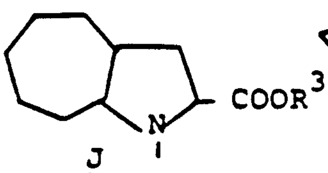
J

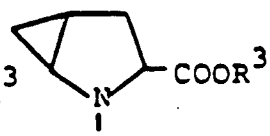
K

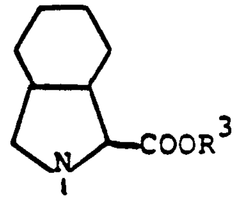
L

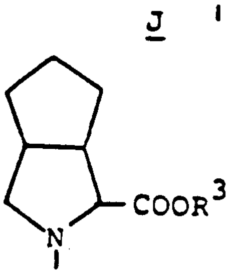
M

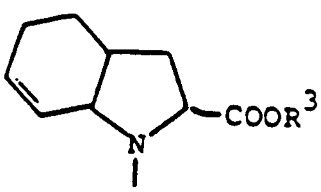
N

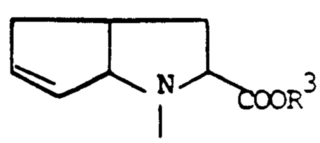
O

Unter Aryl ist hier wie im folgenden vorzugsweise gegebenenfalls substituiertes Phenyl, Biphenylyl oder Naphthyl zu verstehen. Entsprechendes gilt für von Aryl abgeleitete Rest wie Aryloxy, Arylthio. Unter Aroyl wird insbesondere Benzoyl verstanden. Aliphatische Reste können geradkettig oder verzweigt sein.

Unter einem mono- bzw. bicyclischen Heterocyclen-Rest mit 5 bis 7 bzw. 8 bis 10 Ringatomen, wovon 1 bis 2 Ringatome Schwefel oder Sauerstoffatome und/oder wovon 1 bis 4 Ringatome Stickstoffatome darstellen, wird beispielsweise Thienyl, Benzo[b]thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein.

Natürlich vorkommende $\alpha$-Aminosäuren sind z.B. in Houben-Weyl, Methoden der Organischen Chemie, Bd. XV/1 und XV/2 beschrieben.

Falls $R^1$ für eine Seitenkette einer geschützten natürlich vorkommenden $\alpha$-Aminosäure steht, wie z.B. geschütztes Ser, Thr, Asp, Asn, Glu, Gln, Arg,Lys, Hyl, Cys, Orn, Cit, Tyr, Trp, His oder Hyp, sind als Schutzgruppen in der Peptidchemie üblichen Gruppen bevorzugt (vgl. Houben-Weyl, Bd. XV/1 und XV/2). Im Falle, daß $R^1$ die geschützte Lysin-Seitenkette bedeutet, werden die bekannten Amino-Schutzgruppen, insbesondere aber Z, Boc oder ($C_1$-$C_6$)-Alkanoyl bevorzugt. Als O-Schutzgruppen für Tyrosin kommen bevorzugt ($C_1$-$C_6$)-Alkyl, insbesondere Methyl oder Ethyl in Frage.

Insbesondere bevorzugt sind die Verbindungen 2-[N-(1-S-Ethoxycarbonyl-3-phenylpropyl)-S-alanyl]-(1S,3S,5S)-2-azabicyclo[3.3.0]octan-3-carbonsäure (Ramipril), 1-[N-(1-S-Ethoxycarbonyl-3-phenylpropyl)-S-alanyl]-(2S,3aR,7aS)-octahydro[1H]indol-2-carbonsäure (Trandolapril) sowie 2-[N-(1-S-Ethoxycarbonyl-3-phenylpropyl)-S-alanyl]-1.2.3.4-tetrahydroisochinolin-3-S-carbonsäure (Quinapril).

Die ACE-Hemmer der Formel I lassen sich herstellen, indem man ihre Fragmente in einem geeigneten Lösungsmittel gegebenenfalls in Gegenwart einer Base und/oder eines Kupplungshilfsmittels miteinander umsetzt, gegebenenfalls intermediär entstehende ungesättigte Verbindungen, wie Schiffsche Basen, reduziert, zum Schutz reaktiver Gruppen temporär eingeführte Schutzgruppen abspaltet, Verbindungen der Formel I mit freier(en) Carboxylgruppe(n) gegebenenfalls verestert und die erhaltenen Verbindungen gegebenenfalls in ihre physiologisch verträglichen Salze überführt.

In der genannten Weise kann man beispielsweise Verbindungen der Formel V mit Verbindungen der Formel VI umsetzen.

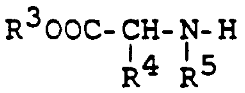

(V)

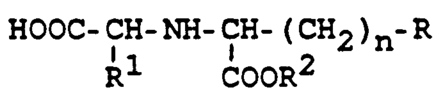

(VI)

Die Umsetzung dieser Verbindungen kann beispielsweise in Analogie zu bekannten Peptidkupplungsverfahren in einem organischen Lösungsmittel wie DMF, $CH_2Cl_2$, DMA in Gegenwart von Kupplungshilfsmitteln, wie Carbodiimiden (z.B. Dicyclohexylcarbodiimid), Diphenylphosphoarylazid, Alkanphosphorsäureanhydriden, Dialkylphosphinsäureanhydriden oder N,N-Succinimidylcarbonate in einem Lösungsmittel wie $CH_3CN$ erfolgen. Aminogruppen in Verbindungen der Formel V können mit Tetraethyldiphosphit aktiviert werden. Die Verbindungen der Formel VI können in Aktivester (z.B. mit 1-Hydroxybenzotriazol), gemischte Anhydride (z.B. mit Chlorameisensäureestern), Azide oder Carbodiimid-Derivate überführt und damit aktiviert werden (vgl. Schröder, Lübke, The Peptides, Band 1, New York 1965, Seiten 76 - 136). Die Reaktion wird vorzugsweise zwischen -20 °C und dem Siedepunkt des Reaktionsgemisches durchgeführt.

Ebenso lassen sich auch Verbindungen der Formel VII mit Verbindungen der Formel VIII unter Bildung von Verbindungen der Formel I umsetzen,

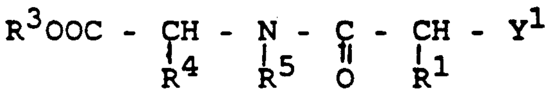

(VII)

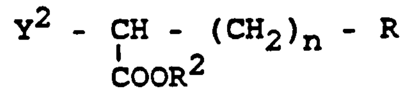

(VIII)

worin entweder $Y^1$ für Amino und $Y^2$ für eine Abgangsgruppe oder $Y^1$ für eine Abgangsgruppe und $Y^2$ für Amino stehen. Geeignete Abgangsgruppen sind z.B. Cl, Br, I, Alkylsulfonyloxy oder Arylsulfonyloxy.

Alkylierungen dieser Art führt man zweckmäßigerweise in Wasser oder einem organischen Lösungsmittel wie einem niedrigen aliphatischen Alkohol (wie Ethanol), Benzylalkohol, Acetonitril, Nitromethan oder Glycolether bei einer Temperatur zwischen -20°C und dem Siedepunkt des Reaktionsgemisches in Gegenwart einer Base wie einem Alkalimetallhydroxid oder einem organischen Amin durch.

Des weiteren lassen sich Verbindungen der Formel IX mit Verbindungen der Formel X kondensieren,

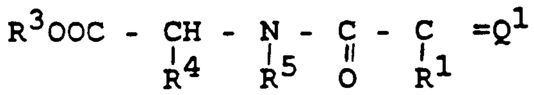

(IX)

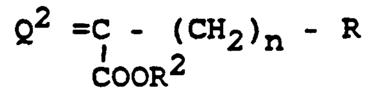

(X)

worin entweder $Q^1$ für Amino + Wasserstoff und $Q^2$ für Oxo steht oder $Q^1$ für Oxo und $Q^2$ für Amino + Wasserstoff steht.

Die Kondensation wird zweckmäßigerweise in Wasser oder einem organischen Lösungsmittel, wie einem niederen aliphatischen Alkohol, bei einer Temperatur zwischen -20°C und dem Siedepunkt des Reaktionsgemisches in Gegenwart eines Reduktionsmittels, wie $NaBH_3CN$, durchgeführt, wobei Verbindungen der Formel I direkt erhalten werden. Man kann aber auch die als Zwischenprodukte entstehenden Schiff'schen Basen oder Enamine gegebenenfalls nach vorheriger Isolierung unter Bildung von Verbindungen der Formel II reduzieren, beispielsweise durch Hydrierung in Gegenwart eines Übergangsmetallkatalysators.

Schließlich führt auch die Umsetzung von Verbindungen der Formel IX ($Q^1$ = H + $NH_2$) mit Verbindungen der Formel XI oder deren Umsetzung mit Verbindungen der Formeln XII und XIII zweckmäßig in Gegenwart einer Base, wie Natriumalkoholat, in einem organischen Lösungsmittel, wie einem niederen Alkohol, bei einer Temperatur zwischen -10°C und dem Siedepunkt des Reaktionsgemisches zu Verbindungen der Formel II (n = 2),

$R^2OOC\text{-}CH=CH\text{-}COR$ (XI)
$OCH\text{-}COOR^2$ (XII)
$R\text{-}CO\text{-}CH_3$ (XIII)

wobei intermediär entstehende Schiff'sche Basen wie oben beschrieben reduziert und eine Carbonylgruppe reduktiv (beispielsweise mit einem komplexen Hydrid) in Methylen überführt werden.

In den obengenannten Formeln V - XIII sind R - $R^5$ und n wie in Formel I definiert. Temporär zum Schutz nicht an der Reaktion beteiligter reaktiver Gruppen eingeführte Schutzgruppen werden nach beendeter Reaktion in an sich bekannter Weise abgespalten (vgl. Schröder, Lübke, loc cit., Seiten 1 - 75 und 246 - 270; Greene, "Protective Groups in Organic Synthesis", New York 1981).

Als Calciumantagonisten kommen die Verbindungen der Formel II

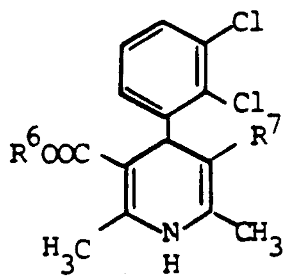

(II)

in Betracht, in welcher $R^6$ Methyl, Ethyl oder Isopropyl und $R^7$ Methoxycarbonyl, Ethoxycarbonyl oder 1,2,4-Oxadiazol-3-yl bedeuten, sowie deren physiologisch verträglichen Salze.

Insbesondere bevorzugt sind

4-(2,3-Dichlorphenyl)-2,6-dimethyl-3-methoxycarbonyl-5-ethoxycarbonyl-1,4-dihydropyridin (Felodipin, Formel II); 4-(2,3-Dichlorphenyl)-2,6-dimethyl-3-(1,2,4-oxadiazol-3-yl)-5-ispropoxycarbonyl-1,4-dihydropyridin (Formel II);
sowie deren physiologisch verträglichen Salze mit Säuren.

Von ganz besonderen Interesse sind die folgenden Kombinationen:
Ramipril + Felodipin oder
Ramipril + 4-(2,3-Dichlorphenyl)-2,6-dimethyl-3-(1,2,4-oxodiazol-5-yl)-5-isopropoxycarbonyl-1,4-dihydropyridin oder
Trandolapril + Felodipin oder
Trandolapril + 4-(2,3-Dichlorphenyl)-2,6-dimethyl-3-(1,2,4-oxadiazol-3-yl)-5-isopropoxycarbonyl-1,4-dihydropyridin oder
Quinapril + Felodipin oder
Quinapril + 4-(2,3-Dichlorphenyl)-2,6-dimethyl-3-(1,2,4-oxodiazol-5-yl)-5-isopropoxycarbonyl-1,4-dihydropyridin oder
sowie jeweils die physiologisch verträglichen Salze der genannten Einzelkomponenten, soweit diese Salze bilden.

Die Erfindung betrifft auch ganz allgemein Erzeugnisse, die enthalten:

a) einen ACE-Hemmer der Formel I oder dessen physiologisch verträgliches Salz und
b) einen Calciumantagonisten oder dessen physiologisch verträgliches Salz

als Kombinationspräparat zur gleichzeitigen, getrennten oder zeitlich abgestuften Anwendung bei der Behandlung des Bluthochdrucks.

Die pharmazeutischen Zubereitungen können beispielsweise hergestellt werden, indem man entweder die Einzelkomponenten als Pulver intensiv mischt, oder indem man die Einzelkomponenten in einem geeigneten Lösungsmittel wie beispielsweise einem niederen Alkohol auflöst und das Lösungsmittel anschließend entfernt.

Das Verhältnis der Wirkstoffe in den erfindungsgemäßen Kombinationen und Zubereitungen beträgt vorzugsweise 1 - 15 Gewichtsteile ACE-Hemmer zu 15 - 1 Gewichtsteilen Calciumantagonist. Die erfindungsgemäßen Kombinationen und Zubereitungen enthalten ingesamt vorzugsweise 0,5 - 99,5 Gew.-%, insbesondere 4 - 96 Gew.-% dieser Wirkstoffe.

Wie oben erwähnt, können die erfindungsgemäßen Zubereitungen und Kombinationen in Arzneimitteln, besonders zur Behandlung von Bluthochdruck, Herzinsuffizienz und coranarer Herzkrankheit verwendet werden.

Die erfindungsgemäßen Zubereitungen und Kombinationen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumkarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträglichen Salze, gewünschtenfalls mit den dafür üblichen Substanzen wie Lösungsvermittler, Emulgatoren, oder weitere Hilfsstoffe in Lösung, Suspension oder Emulsion gebracht. Als Lösungsmittel für die aktiven Kombinationen und die entsprechenden physiologisch verträglichen Salze kommen z.B. in Frage: Wasser, physiologische Kochsalzlösungen oder Alkohole, z.B. Ethanol, Propandiol, oder Glycerin, daneben auch Zuckerlösungen wie Glucose- oder Mannitlösungen oder auch eine Mischung aus den verschiedenen genannten Lösungsmitteln.

Als Salze der Verbindungen der Formel I bis IV kommen, je nach saurer oder basischer Natur dieser Verbindungen, Alkali- oder Erdalkalisalze oder Salze mit physiologisch verträglichen Aminen oder Salze mit anorganischen oder organischen Säuren wie z.B. HCl, HBr, $H_2SO_4$, Maleinsäure, Fumarsäure, Weinsäure, Citronensäure in Frage.

Die folgenden Beispiele dienen zur Erläuterung der vorliegenden Erfindung:

Beispiel 1:

Herstellung eines oralen Kombinationspräparats aus Ramipril und Felodipin
1000 Tabletten, die 2 mg Ramipril und 6 mg Felodipin enthalten, werden wie folgt hergestellt:

| | |
|---|---|
| Ramipril | 2 g |
| Felodipin | 6 g |
| Maisstärke | 140 g |
| Gelatine | 7,5 g |
| Mikrokristalline Cellulose | 2,5 g |
| Magnesiumstearat | 2,5 g |

Die beiden Wirkstoffe werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das so hergestellte Granulat wird zu 1000 Tabletten verpreßt, wobei jede Tablette 2 mg Ramipril und 6 mg Felodipin enthält.

Beispiel 2:

Herstellung eines oralen Kombinationspräparats aus Trandolapril und Felodipin
1000 Tabletten, die 3 mg Trandolapril und 5 mg Felodipin enthalten, werden wie folgt hergestellt:

| | |
|---|---|
| Trandolapril | 3 g |
| Felodipin | 5 g |
| Maisstärke | 130 g |
| Gelatine | 8,0 g |
| Mikrokristalline Cellulose | 2,0 g |
| Magnesiumstearat | 2,0 g |

Die beiden Wirkstoffe werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das so hergestellte Granulat wird zu 1000 Tabletten verpreßt, wobei jede Tablette 3 mg Trandolapril und 5 mg Felodipin enthält.

Beispiel 3:

Herstellung eines oralen Kombinationspräparats aus Quinapril und Felodipin
1000 Tabletten, die 2,5 mg Quinapril und 6 mg Felodipin enthalten, werden wie folgt hergestellt:

| | |
|---|---|
| Quinapril | 2,5 g |
| Felodipin | 5 g |
| Maisstärke | 150 g |
| Gelatine | 7,5 g |
| Mikrokristalline Cellulose | 2,5 g |
| Magnesiumstearat | 2,5 g |

Die beiden Wirkstoffe werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das so hergestellte Granulat wird zu 1000 Tabletten verpreßt, wobei jede Tablette 2,5 mg Quinapril und 5 mg Felodipin enthält.

**Patentansprüche**
**Patentansprüche für folgende Vertragsstaaten : BE, CH, DE, FR, GB, IT, LI, LU, NL, SE**

**1.** Pharmazeutische Zubereitung enthaltend
a) einen Angiotensin-Converting-Enzyme-Hemmer (ACE- (ACEHemmer) der Formel I

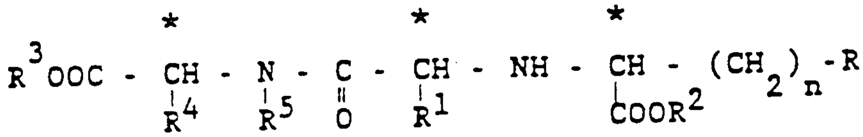

(I)

in welcher

n = 1 oder 2 ist,

R = $(C_1-C_6)$-Alkyl, $(C_2-C_6)$-Alkenyl, $(C_3-C_9)$-Cycloalkyl, Amino-$(C_1-C_4)$-alkyl, $(C_2-C_5)$-Acylamino-$(C_1-C_4)$-alkyl, $(C_7-C_{13})$-Aroylamino-$(C_1-C_4)$-alkyl, $(C_1-C_4)$-Alkoxycarbonylamino-$(C_1-C_4)$-alkyl, $(C_6-C_{12})$-Aryl-$(C_1-C_4)$-alkoxycarbonylamino-$(C_1-C_4)$-alkyl, $(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino und/oder Methylendioxy mono-, di- oder trisubstituiert sein kann, oder 3-Indolyl, insbesondere Methyl, Ethyl, Cyclohexyl, tert. Butoxycarbonylamino-$(C_1-C_4)$-alkyl, Benzoyloxycarbonylamino-$(C_1-C_4)$-alkyl oder Phenyl, das durch Phenyl, $(C_1-C_2)$-Alkyl, ($C_1$ oder $C_2$)-Alkoxy, Hydroxy, Fluor, Chlor, Brom, Amino, $(C_1-C_4)$-Alkylamino, Di-$(C_1-C_4)$alkylamino, Nitro und/ oder Methylendioxy mono- oder disubstituiert oder im Falle von Methoxy, trisubstituiert sein kann, bedeutet.

$R^1$ Wasserstoff oder $(C_1-C_6)$-Alkyl, das gegebenenfalls durch Amino, $(C_1-C_6)$-Acylamino oder Benzoylamino substituiert sein kann, $(C_2-C_6)$-Alkenyl, $(C_3-C_9)$-Cycloalkyl, $(C_5-C_9)$-Cycloalkenyl, $(C_3-C_7)$-Cycloalkyl-$(C_1-C_4)$-alkyl, $(C_6-C_{12})$-Aryl oder teilhydriertes Aryl, das jeweils durch $(C_1-C_4)$-Alkyl, ($C_1$ oder $C_2$)-Alkoxy oder Halogen substituiert sein kann, $(C_6-C_{12})$-Aryl-($C_1$ bis $C_4$)-alkyl oder $(C_7-C_{13})$-Aroyl-$(C_1-C_2)$-alkyl, die beide wie vorstehend definiert im Arylrest substituiert sein können, ein mono- bzw. bicyclischer Heterocyclen-Rest mit 5 bis 7 bzw. 8 bis 10 Ringatome, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten $\alpha$-Aminosäure, insbesondere aber Wasserstoff, $(C_1-C_3)$-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, $(C_1-C_6)$-Alkyl, $(C_2-C_6)$-Alkenyl oder $(C_6-C_{12})$-Aryl-$(C_1-C_4)$-alkyl, insbesondere aber Wasserstoff, $(C_1-C_4)$-Alkyl oder Benzyl bedeuten und

$R^4$ und $R^5$ zusammen mit den diese tragenden Atomen ein Ringsystem aus der Reihe Tetrahydroisochinolin; Decahydroisochinolin; Octahydroindol; Octahydrocyclopenta[b]pyrrol; 2-Azaspiro[4.5]decan; 2-Azaspiro[4.4]nonan; Spiro[-(bicyclo[2.2.1]heptan)-2,3'-pyrrolidin]; Spiro[(bicyclo[2.2.2]-octan)-2,3'-pyrrolidin]; 2-Azatricyclo[4,3,o,$1^{6,9}$]decan; Decahydrocyclohepta[b]pyrrol; Octahydroisoindol; Octahydrocyclopenta[c]pyrrol; 2,3,3a,4,5,7a-Hexahydroindol; 2-Azabicyclo[3.1.0]-hexan und Hexahydrocyclopenta[b]pyrrol, die alle gegebenenfalls substituiert sein können, bilden, oder dessen physiologisch verträgliches Salz

b) einen Calciumantagonisten der Formel II

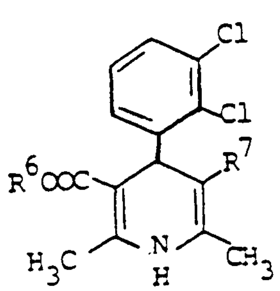

(II)

in welcher

$R^6$ Methyl, Ethyl oder Isopropyl und
$R^7$ Methoxycarbonyl, Ethoxycarbonyl oder 1,2,4-Oxadiazol-3-yl bedeuten, oder dessen physiologisch verträgliches Salz.

**2.** Zubereitung gemäß Anspruch 1, enthaltend 4-(2,3-Dichlorphenyl)-2,6-dimethyl-3-methoxycarbonyl-5-ethoxycarbonyl-1,4-dihydropyridin oder dessen physiologisch verträgliches Salz.

**3.** Zubereitung gemäß Anspruch 1, enthaltend
Ramipril + Felodipin oder
Ramipril + 4-(2,3-Dichlorphenyl)-2,6-dimethyl-3-(1,2,4-oxodiazol-5-yl)-5-isopropoxycarbonyl-1,4-dihydropyridin oder
Trandolapril + Felodipin oder
Trandolapril + 4-(2,3-Dichlorphenyl)-2,6-dimethyl-3-(1,2,4-oxadiazol-5-yl)-5-isopropoxycarbonyl-1,4-dihydropyridin oder
Quinapril + Felodipin oder
Quinapril + 4-(2,3-Dichlor)-2,6-dimethyl-3-(1,2,4-oxodiazol-5-yl)-5-isopropoxycarbonyl-1,4-dihydropyridin oder jeweils die physiologisch verträglichen Salze der genannten Einzelkomponenten, soweit diese Salze bilden.

**4.** Verfahren zur Herstellung einer Zubereitung gemäß Anspruch 1, dadurch gekennzeichnet, daß man
a) einen ACE-Hemmer oder dessen physiologisch verträgliches Salz und
b) einen Calciumantagonisten oder dessen physiologisch verträgliches Salz zusammen mit einem physiologisch annehmbaren Träger und gegebenenfalls weiteren Hilfs- oder Zusatzstoffen in eine geeignete Darreichungsform bringt.

**5.** Erzeugnis, enthaltend
a) einen ACE-Hemmer der Formel (I) gemäß Anspruch 1 oder dessen physiologisch verträgliches Salz und
b) einen Calciumantagonisten der Formel (II) oder dessen physiologisch verträgliches Salz gemäß Anspruch 1.
als Kombinationspräparat zur gleichzeitigen, getrennten oder zeitlich abgestuften Anwendung bei der Behandlung des Bluthochdrucks.

**Patentansprüche für folgende Vertragsstaaten : AT, ES, GR**

**1.** Verfahren zur Herstellung einer pharmazeutischen Zubereitung enthaltend
a) eine wirksame Menge eines Angiotensin-Converting-Enzyme-Hemmers (ACE-Hemmer) der Formel I

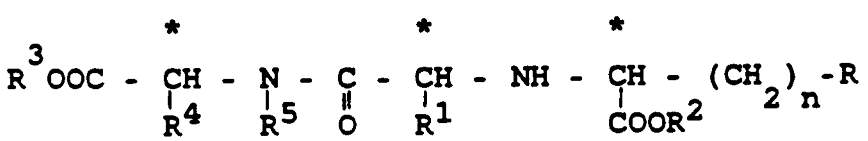

(I)

in welcher

n = 1 oder 2 ist,

R = $(C_1$-$C_6)$-Alkyl, $(C_2$-$C_6)$-Alkenyl, $(C_3$-$C_9)$-Cycloalkyl, Amino-$(C_1$-$C_4)$-alkyl, $(C_2$-$C_5)$-Acylamino-$(C_1$-$C_4)$-alkyl, $(C_7$-$C_{13})$-Aroylamino-$(C_1$-$C_4)$-alkyl, $(C_1$-$C_4)$-Alkoxycarbonylamino-$(C_1$-$C_4)$-alkyl, $(C_6$-$C_{12})$-Aryl-$(C_1$-$C_4)$-alkoxycarbonylamino-$(C_1$-$C_4)$-alkyl, $(C_6$-$C_{12})$-Aryl, das durch $(C_1$-$C_4)$-Alkyl, $(C_1$-$C_4)$-Alkoxy, Hydroxy, Halogen, Nitro, Amino, $(C_1$-$C_4)$-Alkylamino, Di-$(C_1$-$C_4)$-alkylamino und/oder Methylendioxy mono-, di- oder tri-substituiert sein kann, oder 3-Indolyl, insbesondere Methyl, Ethyl, Cyclohexyl, tert. Butoxycarbonylamino-$(C_1$-$C_4)$-alkyl, Benzoyloxycarbonylamino-$(C_1$-$C_4)$-alkyl oder Phenyl, das durch Phenyl, $(C_1$-$C_2)$-Alkyl, $(C_1$ oder $C_2)$-Alkoxy, Hydroxy, Fluor, Chlor, Brom, Amino, $(C_1$-$C_4)$-Alkylamino, Di-$(C_1$-$C_4)$alkylamino, Nitro und/ oder Methylendioxy mono- oder disubstituiert

oder im Falle von Methoxy, trisubstituiert sein kann, bedeutet.

$R^1$ Wasserstoff oder ($C_1$-$C_6$)-Alkyl, das gegebenenfalls durch Amino, ($C_1$-$C_6$)-Acylamino oder Benzoylamino substituiert sein kann, ($C_2$-$C_6$)-Alkenyl, ($C_3$-$C_9$)-Cycloalkyl, ($C_5$-$C_9$)-Cycloalkenyl, ($C_3$-$C_7$)-Cycloalkyl-($C_1$-$C_4$)-alkyl, ($C_6$-$C_{12}$)-Aryl oder teilhydriertes Aryl, das jeweils durch ($C_1$-$C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)-alkyl, die beide wie vorstehend definiert im Arylrest substituiert sein können, ein mono- bzw. bicyclischer Heterocyclen-Rest mit 5 bis 7 bzw. 8 bis 10 Ringatome, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten $\alpha$-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, ($C_1$-$C_6$)-Alkyl, ($C_2$-$C_6$)-Alkenyl oder ($C_6$-$C_{12}$)-Aryl-($C_1$-$C_4$)-alkyl, insbesondere aber Wasserstoff, ($C_1$-$C_4$)-Alkyl oder Benzyl bedeuten und

$R^4$ und $R^5$ zusammen mit den diese tragenden Atomen ein Ringsystem aus der Reihe Tetrahydroisochinolin; Decahydroisochinolin; Octahydroindol; Octahydrocyclopenta[b]pyrrol; 2-Azaspiro[4.5]decan; 2-Azaspiro[4.4]nonan; Spiro[-(bicyclo[2.2.1]heptan)-2,3'-pyrrolidin]; Spiro[(bicyclo[2.2.2]-octan)-2,3'-pyrrolidin]; 2-Azatricyclo[4,3,o,$1^{6,9}$]decan; Decahydrocyclohepta[b]pyrrol; Octahydroisoindol; Octahydrocyclopenta[c]pyrrol; 2,3,3a,4,5,7a-Hexahydroindol; 2-Azabicyclo[3.1.0]-hexan und Hexahydrocyclopenta[b]pyrrol, die alle gegebenenfalls substituiert sein können, bilden, oder dessen physiologisch verträgliches Salz

b) einen Calciumantagonisten der Formel II

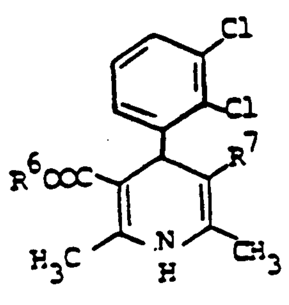

(II)

in welcher
$R^6$ Methyl, Ethyl oder Isopropyl und
$R^7$ Methoxycarbonyl, Ethoxycarbonyl oder 1,2,4-Oxadiazol-3-yl bedeuten, oder dessen physiologisch verträgliches Salz,
dadurch gekennzeichnet, daß man
a) einen ACE-Hemmer oder dessen physiologisch verträgliches Salz und
b) einen Calciumantogonisten oder dessen physiologisch verträgliches Salz
zusammen mit einem physiologisch annehmbaren Träger und gegebenenfalls weiteren Hilfs- oder Zusatzstoffen in eine geeignete Darreichungsform bringt.

2. Verfahren gemäß Anspruch 1, dadurch gekennzeichnet, daß 4-(2,3-Dichlorphenyl)-2,6-dimethyl-3-methoxycarbonyl-5-ethoxycarbonyl-1,4-dihydropyridin oder dessen physiologisch verträgliches Salz eingesetzt wird.

3. Verfahren gemäß Anspruch 1, dadurch gekennzeichnet, daß Ramipril + Felodipin oder
Ramipril + 4-(2,3-Dichlorphenyl)-2,6-dimethyl-3-(1,2,4-oxodiazol-5-yl)-5-isopropoxycarbonyl-1,4-dihydropyridin oder
Trandolapril + Felodipin oder
Trandolapril + 4-(2,3-Dichlorphenyl)-2,6-dimethyl-3-(1,2,4-oxadiazol-3-yl-5-isopropoxycarbonyl-1,4-dihydropyridon oder

Quinapril + Felodipin oder
Quinapril + 4-(2,3-Dichlorphenyl)-2,6-dimethyl-3-(1,2,4-oxodiazol-5-yl)-5-isopropoxycarbonyl-1,4-dihydropyridin oder
jeweils deren physiologisch verträglichen Salze, soweit diese Salze bilden, eingesetzt werden.

**4.** Verfahren zur Herstellung eines Erzeugnisses, enthaltend
a) einen ACE-Hemmer der Formel I gemäß Anspruch 1 oder dessen physiologisch verträgliches Salz und
b) einen Calciumantagonisten oder dessen physiologisch verträgliches Salz gemäß Anspruch 1
als Kombinationspräparat zur gleichzeitigen, getrennten oder zeitlich abgestuften Anwendung bei der Behandlung des Bluthochdrucks, dadurch gekennzeichnet, daß die Wirkstoffe jeweils zusammen mit einem physiologisch annehmbaren Träger und gegebenenfalls weiteren Zusatz-oder Hilfsstoffen in eine geeignete Darreichungsform gebracht werden.

## Claims

**Claims for the following Contracting States : BE, CH, DE, FR, GB, IT, LI, LU, NL, SE**

**1.** A pharmaceutical composition containing
a) an angiotensin-converting enzyme inhibitor (ACE inhibitor) of the formula I

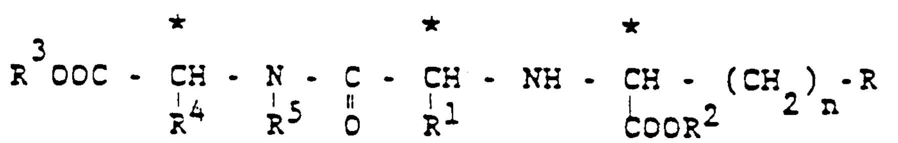

(I)

in which

n = 1 or 2,

R is $(C_1-C_6)$-alkyl, $(C_2-C_6)$-alkenyl, $(C_3-C_9)$-cycloalkyl, amino-$(C_1-C_4)$-alkyl, $(C_2-C_5)$-acylamino-$(C_1-C_4)$-alkyl, $(C_7-C_{13})$-aroylamino-$(C_1-C_4)$-alkyl, $(C_1-C_4)$-alkoxycarbonylamino-$(C_1-C_4)$-alkyl, $(C_6-C_{12})$-aryl-$(C_1-C_4)$-alkoxycarbonylamino-$(C_1-C_4)$-alkyl, $(C_6-C_{12})$-aryl, which can be mono-, di- or tri-substituted by $(C_1-C_4)$-alkyl, $(C_1-C_4)$-alkoxy, hydroxy, halogen, nitro, amino, $(C_1-C_4)$-alkylamino, di-$(C_1-C_4)$-alkylamino and/or methylenedioxy, or 3-indolyl, especially methyl, ethyl, cyclohexyl, tert.-butoxycarbonylamino-$(C_1-C_4)$-alkyl, benzoyloxycarbonyl amino-$(C_1-C_4)$-alkyl or phenyl, which can be mono- or di-substituted or, in the case of methoxy, trisubstituted by phenyl, $(C_1-C_2)$-alkyl, ($C_1$ or $C_2$)-alkoxy, hydroxy, fluoro, chloro, bromo, amino, $(C_1-C_4)$-alkylamino, di-$(C_1-C_4)$-alkylamino, nitro and/or methylenedioxy,

$R^1$ is hydrogen or $(C_1-C_6)$-alkyl, which can be optionally substituted by amino, $(C_1-C_6)$-acylamino or benzoylamino, $(C_2-C_6)$-alkenyl, $(C_3-C_9)$-cycloalkyl, $(C_5-C_9)$-cycloalkenyl, $(C_3-C_7)$-cycloalkyl-$(C_1-C_4)$-alkyl, $(C_6-C_{12})$-aryl or partially hydrogenated aryl, which can each be substituted by $(C_1-C_4)$-alkyl, ($C_1$ or $C_2$)-alkoxy or halogen, $(C_6-C_{12})$-aryl-($C_1$ to $C_4$)-alkyl or $(C_7-C_{13})$-aroyl-$(C_1-C_2)$-alkyl, which can both be substituted as defined previously in the aryl radical, a mono- or bi-cyclic heterocyclic radical with 5 to 7 or 8 to 10 ring atoms respectively, of which 1 to 2 ring atoms represent sulfur or oxygen atoms and/or 1 to 4 ring atoms represent nitrogen atoms, or a side chain of a naturally occurring, optionally protected $\alpha$-amino acid, but especially hydrogen, $(C_1-C_3)$-alkyl, ($C_2$ or $C_3$)-alkenyl, the optionally protected side chain of lysine, benzyl,4-methoxybenzyl, 4-ethoxybenzyl, phenethyl, 4-aminobutyl or benzoylmethyl,

$R^2$ and $R^3$ are the same or different and are hydrogen, $(C_1-C_6)$-alkyl, $(C_2-C_6)$-alkenyl or $(C_6-C_{12})$-aryl-$(C_1-C_4)$-alkyl, but especially hydrogen, $(C_1-C_4)$-alkyl or benzyl, and

$R^4$ and $R^5$ together with the atoms carrying them form a ring system from the series tetrahydroisoquinoline; decahydroisoquinoline; octahydroindole; octahydrocyclopenta[b]pyrrole; 2-azaspiro[4,5]decane; 2-azaspiro[4,4]nonane; spiro[(bicyclo[2,2,1]heptane)-2,3'-pyrrolidine]; spiro[(bicyclo)-[2,2,2]octane)-2,3'-pyrrolidine]; 2-azatricyclo-[4,3,0,$1^{6,9}$]decane ; decahydrocyclohepta[b]pyrrole; octahydroisoindole; octahydrocyclopenta[c]pyrrole; 2,3,3a,4,5,7a-hexahydroindole; 2-azabicyclo[3,1,0]-hexane and hexahydrocyclopenta[b]pyrrole, which can all be optionally substituted, or a physiologically acceptable salt thereof and

b) a calcium antagonist of the formula II

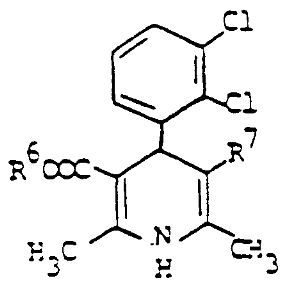

(II)

in which

$R^6$ is methyl, ethyl or isopropyl and

$R^7$ is methoxycarbonyl, ethoxycarbonyl or 1,2,4-oxadiazol-3-yl, or a physiologically acceptable salt thereof.

**2.** A composition as claimed in claim 1, containing 4-(2,3-dichlorophenyl)-2,6-dimethyl-3-methoxycarbonyl-5-ethoxycarbonyl-1,4-dihydropyridine or a physiologically acceptable salt thereof.

**3.** A composition as claimed in claim 1, containing
ramipril + felodipine or
ramipril + 4-(2,3-dichlorophenyl)-2,6-dimethyl-3-(1,2,4-oxodiazol-5-yl)-5-isopropoxycarbonyl-1,4-dihydropyridine or
trandolapril + felodipine or
trandolapril + 4-(2,3-dichlorophenyl)-2,6-dimethyl-3-(1,2,4-oxadiazol-5-yl)-5-isopropoxycarbonyl-1,4-dihydropyridine or
quinapril + felodipine or
quinapril + 4-(2,3-dichloro)-2,6-dimethyl-3-(1,2,4-oxodiazol-5-yl)-5-isopropoxycarbonyl-1,4-dihydropyridine [sic] or in each case the physiologically acceptable salts of the above single components, in so far as these form salts.

**4.** A process for the preparation of a composition as claimed in claim 1, wherein
a) an ACE inhibitor or a physiologically acceptable salt thereof and
b) a calcium antagonist or a physiologically acceptable salt thereof
are combined in a suitable form for administration with a physiologically acceptable carrier and optionally further auxiliaries or additives.

**5.** A product, containing
a) an ACE inhibitor of the formula (I) as claimed in claim 1, or a physiologically acceptable salt thereof and
b) a calcium antagonist of the formula (II) or a physiologically acceptable salt thereof as claimed in claim 1
as a combination preparation for simultaneous, separate or periodic regulated use in the treatment of high blood pressure.

**Claims for the following Contracting States : AT, ES, GR**

**1.** A process for the preparation of a pharmaceutical composition containing
a) an effective amount of an angiotensin-converting enzyme inhibitor (ACE inhibitor) of the formula I

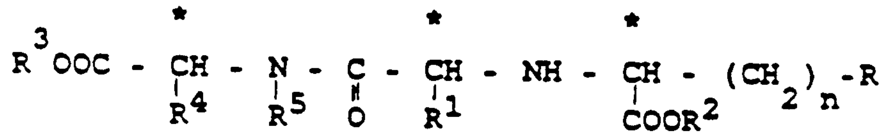

(I)

in which

n = 1 or 2,

R is $(C_1-C_6)$-alkyl, $(C_2-C_6)$-alkenyl, $(C_3-C_9)$-cycloalkyl, amino-$(C_1-C_4)$-alkyl, $(C_2-C_5)$-acylamino-$(C_1-C_4)$-alkyl, $(C_7-C_{13})$-aroylamino-$(C_1-C_4)$-alkyl, $(C_1-C_4)$-alkoxycarbonylamino-$(C_1-C_4)$-alkyl, $(C_6-C_{12})$-aryl-$(C_1-C_4)$-alkoxycarbonylamino-$(C_1-C_4)$-alkyl, $(C_6-C_{12})$-aryl, which can be mono-, di- or trisubstituted by $(C_1-C_4)$-alkyl, $(C_1-C_4)$-alkoxy, hydroxy, halogen, nitro, amino, $(C_1-C_4)$-alkylamino, di-$(C_1-C_4)$-alkylamino and/or methylenedioxy, or 3-indolyl, especially methyl, ethyl, cyclohexyl, tert.-butoxycarbonylamino-$(C_1-C_4)$-alkyl, benzoyloxycarbonyl amino-$(C_1-C_4)$-alkyl or phenyl, which can be mono- or di-substituted or, in the case of methoxy, trisubstituted by phenyl, $(C_1-C_2)$-alkyl, ($C_1$ or $C_2$)-alkoxy, hydroxy, fluoro, chloro, bromo, amino, $(C_1-C_4)$-alkylamino, di-$(C_1-C_4)$-alkylamino, nitro and/or methylenedioxy,

$R^1$ is hydrogen or $(C_1-C_6)$-alkyl, which can be optionally substituted by amino, $(C_1-C_6)$-acylamino or benzoylamino, $(C_2-C_6)$-alkenyl, $(C_3-C_9)$-cycloalkyl, $(C_5-C_9)$-cycloalkenyl, $(C_3-C_7)$-cycloalkyl-$(C_1-C_4)$-alkyl, $(C_6-C_{12})$-aryl or partially hydrogenated aryl, which can each be substituted by $(C_1-C_4)$-alkyl, ($C_1$ or $C_2$)-alkoxy or halogen, $(C_6-C_{12})$-aryl-($C_1$ to $C_4$)-alkyl or $(C_7-C_{13})$-aroyl-$(C_1-C_2)$-alkyl, which can both be substituted as defined previously in the aryl radical, a mono- or bi-cyclic heterocyclic radical with 5 to 7 or 8 to 10 ring atoms respectively, of which 1 to 2 ring atoms represent sulfur or oxygen atoms and/or 1 to 4 ring atoms represent nitrogen atoms, or a side chain of a naturally occurring, optionally protected $\alpha$-amino acid, but especially hydrogen, $(C_1-C_3)$-alkyl, ($C_2$ or $C_3$)-alkenyl, the optionally protected side chain of lysine, benzyl,4-methoxybenzyl, 4-ethoxybenzyl, phenethyl, 4-aminobutyl or benzoylmethyl,

$R^2$ and $R^3$ are the same or different and are hydrogen, $(C_1-C_6)$-alkyl, $(C_2-C_6)$-alkenyl or $(C_6-C_{12})$-aryl-$(C_1-C_4)$-alkyl, but especially hydrogen, $(C_1-C_4)$-alkyl or benzyl, and

$R^4$ and $R^5$ together with the atoms carrying them form a ring system from the series tetrahydroisoquinoline; decahydroisoquinoline; octahydroindole; octahydrocyclopenta[b]pyrrole; 2-azaspiro[4,5]decane; 2-azaspiro[4,4]nonane; spiro[(bicyclo[2,2,1]heptane)-2,3'-pyrrolidine]; spiro[(bicyclo)-[2,2,2]octane)-2,3'-pyrrolidine]; 2-azatricyclo-[4,3,0,$1^{6,9}$]decane; decahydrocyclohepta[b]pyrrole; octahydroisoindole; octahydrocyclopenta[c]pyrrole; 2,3,3a,4,5,7a-hexahydroindole; 2-azabicyclo[3,1,0]-hexane and hexahydrocyclopenta[b]pyrrole, which can all be optionally substituted, or a physiologically acceptable salt thereof and

b) a calcium antagonist of the formula II

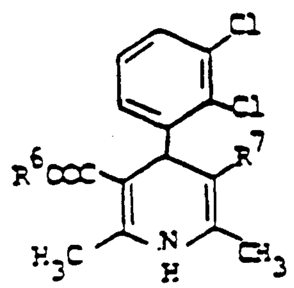

(II)

in which

$R^6$ is methyl, ethyl or isopropyl and

$R^7$ is methoxycarbonyl, ethoxycarbonyl or 1,2,4-oxadiazol-3-yl, or a physiologically acceptable

salt thereof, which comprises combining

a) an ACE inhibitor or a physiologically acceptable salt thereof and

b) a calcium antagonist or a physiologically acceptable salt thereof

in a suitable form for administration with a physiologically acceptable carrier and optionally further auxiliaries or additives.

**2.** The process as claimed in claim 1, wherein 4-(2,3-dichlorophenyl)-2,6-dimethyl-3-methoxycarbonyl-5-ethoxycarbonyl-1,4-dihydropyridine or a physiologically acceptable salt thereof is employed.

**3.** The process as claimed in claim 1, wherein
ramipril + felodipine or
ramipril + 4-(2,3-dichlorophenyl)-2,6-dimethyl-3-(1,2,4-oxodiazol-5-yl)-5-isopropoxycarbonyl-1,4-dihydropyridine or
trandolapril + felodipine or
trandolapril + 4-(2,3-dichlorophenyl)-2,6-dimethyl-3-(1,2,4-oxadiazol-3-yl)-5-isopropoxycarbonyl-1,4-dihydropyridone [sic] or
quinapril + felodipine or
quinapril + 4-(2,3-dichlorophenyl)-2,6-dimethyl-3-(1,2,4-oxodiazol-5-yl)-5-isopropoxycarbonyl-1,4-dihydropyridine or in each case the physiologically acceptable salts thereof, in so far as these form salts, are employed.

**4.** A process for the preparation of a product, containing

a) an ACE inhibitor of the formula I as claimed in claim 1 or a physiologically acceptable salt thereof and

b) a calcium antagonist or a physiologically acceptable salt thereof as claimed in claim 1

as a combination preparation for simultaneous, separate or periodic regulated use in the treatment of high blood pressure, wherein the active agents are combined in each case in a suitable form for administration with a physiologically acceptable carrier and optional further additives or auxiliaries.

**Revendications**

**Revendications pour les Etats contractants suivants : BE, CH, DE, FR, GB, IT, LI, LU, NL, SE**

**1.** Préparation pharmaceutique comprenant:

a) un inhibiteur de l'enzyme de conversion de l'angiotensine (inhibiteur de l'ACE) de formule I

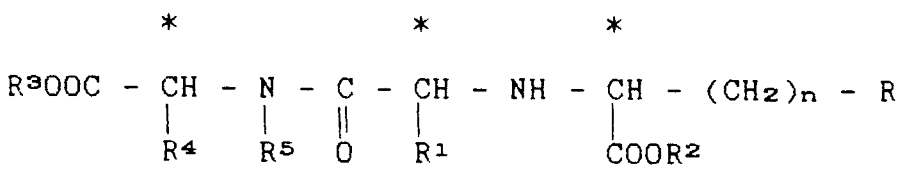

(I)

dans laquelle

n est égal à 1 ou 2,

R représente un groupe alkyle en $C_1$-$C_6$, alcényle en $C_2$-$C_6$, cycloalkyle en $C_3$-$C_9$, (alkyle en $C_1$-$C_4$)amino, (acylamino en $C_2$-$C_5$)(alkyle en $C_1$-$C_4$), (aroylamino en $C_7$-$C_{13}$)(alkyle en $C_1$-$C_4$), (alcoxycarbonylamino en $C_1$-$C_4$)(alkyle en $C_1$-$C_4$), (aryle en $C_8$-$C_{12}$)(alcoxycarbonylamino en $C_1$-$C_4$)-(alkyle en $C_1$-$C_4$), aryle en $C_6$-$C_{12}$, qui peut être mono-, di- ou trisubstitué par un groupe alkyle en $C_1$-$C_4$, un groupe alcoxy en $C_1$-$C_4$, un groupe hydroxy, un atome d'halogène, un groupe nitro, un groupe amino, un groupe alkylamino en $C_1$-$C_4$, un groupe di(alkylamino en $C_1$-$C_4$)
et/ou un groupe méthylènedioxy, ou 3-indolyle,
en particulier un groupe méthyle, éthyle, cyclohexyle, tert.-butoxycarbonylamino(alkyle en $C_1$-$C_4$) ou phényle, qui peut être mono- ou disubstitué ou, dans le cas du groupe méthoxy, trisubstitué par un groupe phényle, un groupe alkyle en $C_1$-$C_2$, un groupe alcoxy en $C_1$ ou $C_2$, un groupe hydroxy, un atome de fluor, un atome de chlore, un atome de brome, un groupe amino, un groupe alkylamino en $C_1$-$C_4$, un groupe di(alkylamino en $C_1$-$C_4$), un groupe nitro et/ou un groupe méthylènedioxy,
$R^1$ représente un atome d'hydrogène ou un groupe alkyle en $C_1$-$C_6$, qui peut éventuellement être substitué par un groupe amino, un groupe acylamino en $C_1$-$C_6$ ou un groupe benzoylamino, alcényle

en $C_2$-$C_6$, cycloalkyle en $C_3$-$C_9$, cycloalcényle en $C_5$-$C_9$, (cycloalkyle en $C_3$-$C_7$)(alkyle en $C_1$-$C_4$), aryle en $C_6$-$C_{12}$ ou aryle partiellement hydrogéné, dont chacun peut être substitué par un groupe alkyle en $C_1$-$C_4$, un groupe alcoxy en $C_1$ ou $C_2$ ou un atome d'halogène, (aryle en $C_8$-$C_{12}$)(alkyle en $C_1$ à $C_4$) ou (aroyle en $C_7$-$C_{13}$)(alkyle en $C_1$-$C_2$) qui peuvent tous les deux être substitués comme défini précédemment pour le radical aryle, un radical d'hétérocycle, mono- ou bicyclique, ayant de 5 à 7, respectivement de 8 à 10 atomes dans le cycle parmi lesquels 1 à 2 atomes sont des atomes de soufre ou d'oxygène et/ou 1 à 4 atomes sont des atomes d'azote, ou une chaîne latérale d'un $\alpha$-aminoacide naturel, éventuellement protégé,

et en particulier un atome d'hydrogène, un groupe alkyle en $C_1$-$C_3$, un groupe alcényle en $C_2$ ou $C_3$, la chaîne latérale, éventuellement protégée, de la lysine, un groupe benzyle, un groupe 4-méthoxybenzyle, un groupe 4-éthoxybenzyle, un groupe phénétyle, un groupe 4-aminobutyle ou un groupe benzoylméthyle,

$R^2$ et $R^3$ sont des radicaux, identiques ou différents, représentant un atome d'hydrogène, un groupe alkyle en $C_1$-$C_6$, alcényle en $C_2$-$C_6$ ou (aryle en $C_6$-$C_{12}$)(alkyle en $C_1$-$C_4$),

et en particulier un atome d'hydrogène, un groupe alkyle en $C_1$-$C_4$ ou un groupe benzyle, et

$R^4$ et $R^5$ constituent, conjointement avec les atomes qui les portent, un système cyclique appartenant aux séries

tétrahydroisoquinoline, décahydroisoquinoline, octahydroindole, octahydrocyclopenta[b]pyrrole, 2-azaspiro[4.5]décane, 2-azaspiro[4.4]nonane, spiro[(bicyclo[2.2.1]heptane)-2,3'pyrrolidine], spiro[-(bicyclo[2.2.2]octane)-2,3'pyrrolidine], 2-azatricyclo[4,3,o,$1^{6,9}$]décane, décahydrocyclohepta[b]pyrrole, octahydroisoindole, octahydrocyclopenta[c]pyrrole, 2,3,3a,4,5,7a-hexahydroindole, 2-azabicyclo-[3.1.0]hexane et hexahydrocyclopenta[b]pyrrole, ces composés pouvant tous être éventuellement substitués,

ou un sel physiologiquement acceptable de celui-ci,

b) un antagoniste du calcium de formule II:

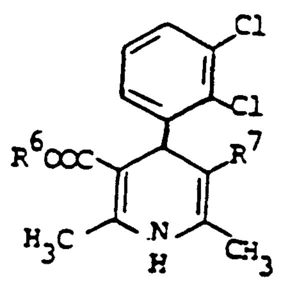

(II)

dans laquelle

$R^6$ représente un groupe méthyle, éthyle ou isopropyle, et

$R^7$ représente un groupe méthoxycarbonyle, éthoxycarbonyle ou 1,2,4-oxadiazol-3-yle,

ou un sel physiologiquement acceptable de celui-ci.

2. Préparation selon la revendication 1, comportant de la 4-(2,3-dichlorophényl)-2,6-diméthyl-3-méthoxycarbonyl-5-éthoxycarbonyl-1,4-dihydropyridine ou un sel physiologiquement acceptable de celle-ci.

3. Préparation selon la revendication 1, comprenant les produits:

ramipril + félodipine ou

ramipril + 4-(2,3-dichlorophényl)-2,6-diméthyl-3-(1,2,4-oxodiazol-5-yl)-5-isopropoxycarbonyl-1,4-dihydropyridine ou

trandolapril + félodipine ou

trandolapril + 4-(2,3-dichlorophényl)-2,6-diméthyl-3-(1,2,4-oxadiazol-5-yl)-5-isopropoxycarbonyl-1,4-dihydropyridine ou

quinapril + félodipine ou

quinapril + 4-(2,3-dichloro)-2,6-diméthyl-3-(1,2,4-oxodiazol-5-yl)-5-isopropoxycarbonyl-1,4-dihydropyridine ou

des sels physiologiquement acceptables de chacun des composants individuels cités, dans la mesure où ces sels existent.

**4.** Procédé pour la fabrication d'une préparation selon la revendication 1, caractérisé en ce que l'on met:
a) un inhibiteur de l'ACE ou un sel physiologiquement acceptable de celui-ci, et
b) un antagoniste du calcium ou un sel physiologiquement acceptable de celui-ci,
conjointement avec un véhicule physiologiquement acceptable et, le cas échéant, d'autres adjuvants et additifs,
sous une forme de présentation appropriée.

**5.** Produit comprenant:
a) un inhibiteur de l'ACE de formule (I) selon la revendication 1 ou un sel physiologiquement acceptable de celui-ci, et
b) un antagoniste du calcium de formule (II) ou un sel physiologiquement acceptable de celui-ci, selon la revendication 1,
en tant que préparation d'une association à utiliser simultanément, séparément ou graduellement, au cours du traitement de l'hypertension artérielle.

**Revendications pour les Etats contractants suivants : AT, ES, GR**

**1.** Procédé pour la fabrication d'une préparation pharmaceutique comprenant:
a) une quantité efficace d'un inhibiteur de l'enzyme de conversion de l'angiotensine (inhibiteur de l'ACE) de formule I

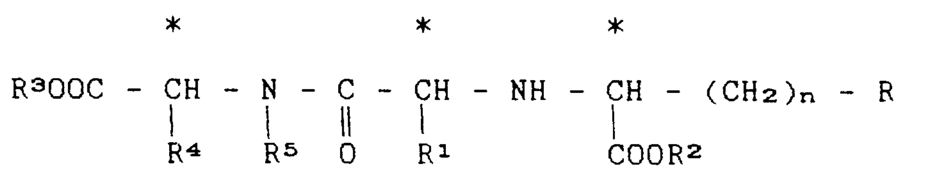

(I)

dans laquelle
n est égal à 1 ou 2,
R représente un groupe alkyle en $C_1$-$C_6$, alcényle en $C_2$-$C_6$, cycloalkyle en $C_3$-$C_9$, (alkyle en $C_1$-$C_4$)amino, (acylamino en $C_2$-$C_5$)(alkyle en $C_1$-$C_4$), (aroylamino en $C_7$-$C_{13}$)(alkyle en $C_1$-$C_4$), (alcoxycarbonylamino en $C_1$-$C_4$)(alkyle en $C_1$-$C_4$), (aryle en $C_6$-$C_{12}$)(alcoxycarbonylamino en $C_1$-$C_4$)-(alkyle en $C_1$-$C_4$), aryle en $C_6$-$C_{12}$, qui peut être mono-, di- ou trisubstitué par un groupe alkyle en $C_1$-$C_4$, un groupe alcoxy en $C_1$-$C_4$, un groupe hydroxy, un atome d'halogène, un groupe nitro, un groupe amino, un groupe alkylamino en $C_1$-$C_4$, un groupe di(alkylamino en $C_1$-$C_4$) et/ou un groupe méthylènedioxy, ou 3-indolyle,
en particulier un groupe méthyle, éthyle, cyclohexyle, tert.-butoxycarbonylamino(alkyle en $C_1$-$C_4$), benzoyloxycarbonylamino(alkyle en $C_1$-$C_4$) ou phényle, qui peut être mono- ou disubstitué ou, dans le cas du groupe méthoxy, trisubstitué par un groupe phényle, un groupe alkyle en $C_1$-$C_2$, un groupe alcoxy en $C_1$ ou $C_2$, un groupe hydroxy, un atome de fluor, un atome de chlore, un atome de brome, un groupe amino, un groupe alkylamino en $C_1$-$C_4$, un groupe di(alkylamino en $C_1$-$C_4$), un groupe nitro et/ou un groupe méthylènedioxy,
$R^1$ représente un atome d'hydrogène ou un groupe alkyle en $C_1$-$C_6$, qui peut éventuellement être substitué par un groupe amino, un groupe acylamino en $C_1$-$C_6$ ou un groupe benzoylamino, alcényle en $C_2$-$C_6$, cycloalkyle en $C_3$-$C_8$, cycloalcényle en $C_5$-$C_8$, (cycloalkyle en $C_3$-$C_7$)(alkyle en $C_1$-$C_4$), aryle en $C_6$-$C_{12}$ ou aryle partiellement hydrogéné, dont chacun peut être substitué par un groupe alkyle en $C_1$-$C_4$, un groupe alcoxy en $C_1$ ou $C_2$ ou un atome d'halogène, (aryle en $C_6$-$C_{12}$)(alkyle en $C_1$ à $C_4$) ou (aroyle en $C_7$-$C_{13}$)(alkyle en $C_1$-$C_2$) qui peuvent tous les deux être substitués comme défini précédemment pour le radical aryle, un radical d'hétérocycle, mono- ou bicyclique, ayant de 5 à 7, respectivement de 8 à 10 atomes dans le cycle parmi lesquels 1 à 2 atomes sont des atomes de soufre ou d'oxygène et/ou 1 à 4 atomes sont des atomes d'azote, ou une chaîne latérale d'un α-aminoacide naturel, éventuellement protégé,
et en particulier un atome d'hydrogène, un groupe alkyle en $C_1$-$C_3$, un groupe alcényle en $C_2$ ou $C_3$,

la chaîne latérale, éventuellement protégée, de la lysine, un groupe benzyle, un groupe 4-méthoxybenzyle, un groupe 4-éthoxybenzyle, un groupe phénétyle, un groupe 4-aminobutyle ou un groupe benzoylméthyle,
$R^2$ et $R^3$ sont des radicaux, identiques ou différents, représentant un atome d'hydrogène, un groupe alkyle en $C_1$-$C_6$, alcényle en $C_2$-$C_6$ ou (aryle en $C_8$-$C_{12}$)(alkyle en $C_1$-$C_4$),
et en particulier un atome d'hydrogène, un groupe alkyle en $C_1$-$C_4$ ou un groupe benzyle, et
$R^4$ et $R^5$ constituent, conjointement avec les atomes qui les portent, un système cyclique appartenant aux séries
tétrahydroisoquinoline, décahydroisoquinoline, octahydroindole, octahydrocyclopenta[b]pyrrole, 2-azaspiro[4.5]décane, 2-azaspiro[4.4]nonane, spiro[(bicyclo[2.2.1]heptane)-2,3'pyrrolidine], spiro[-(bicyclo[2.2.2]octane)-2,3'pyrrolidine], 2-azatricyclo[4,3,o,$1^{6,9}$]décane, décahydrocyclohepta[b]pyrrole, octahydroisoindole, octahydrocyclopenta[c]pyrrole, 2,3,3a,4,5,7a-hexahydroindole, 2-azabicyclo-[3.1.0]hexane et hexahydrocyclopenta[b]pyrrole, ces composés pouvant tous être éventuellement substitués,
ou un sel physiologiquement acceptable de celui-ci,
b) un antagoniste du calcium de formule II:

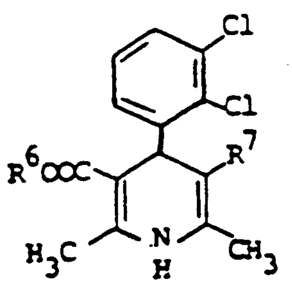

(II)

dans laquelle
$R^6$ représente un groupe méthyle, éthyle ou isopropyle, et
$R^7$ représente un groupe méthoxycarbonyle, éthoxycarbonyle ou 1,2,4-oxadiazol-3-yle,
ou un sel physiologiquement acceptable de celui-ci,
caractérisé en ce que l'on met:

a) un inhibiteur de l'ACE ou un sel physiologiquement acceptable de celui-ci, et
b) un antagoniste du calcium ou un sel physiologiquement acceptable de celui-ci,

conjointement avec un véhicule physiologiquement acceptable et, le cas échéant, d'autres adjuvants et additifs,
sous une forme de présentation appropriée.

**2.** Procédé selon la revendication 1, caractérisé en ce qu'on utilise de la 4-(2,3-dichlorophényl)-2,6-diméthyl-3-méthoxycarbonyl-5-éthoxycarbonyl-1,4-dihydropyridine ou un sel physiologiquement acceptable de celle-ci.

**3.** Procédé selon la revendication 1, caractérisé en ce qu'on utilise les produits:
ramipril + félodipine ou
ramipril + 4-(2,3-dichlorophényl)-2,6-diméthyl-3-(1,2,4-oxodiazol-5-yl)-5-isopropoxycarbonyl-1,4-dihydropyridine ou
trandolapril + félodipine ou
trandolapril + 4-(2,3-dichlorophényl)-2,6-diméthyl-3-(1,2,4-oxadiazol-3-yl)-5-isopropoxycarbonyl-1,4-dihydropyridone ou
quinapril + félodipine ou
quinapril + 4-(2,3-dichlorophényl)-2,6-diméthyl-3-(1,2,4-oxodiazol-5-yl)-5-isopropoxycarbonyl-1,4-dihydropyridine ou
des sels physiologiquement acceptables de chacun des composants individuels cités, dans la mesure où ces sels existent.

**4.** Procédé pour la fabrication d'un produit comprenant:

a) un inhibiteur de l'ACE de formule (I) selon la revendication 1 ou un sel physiologiquement acceptable de celui-ci, et

b) un antagoniste du calcium ou un sel physiologiquement acceptable de celui-ci, selon la revendication 1,

en tant que préparation d'une association à utiliser simultanément, séparément ou graduellement, au cours du traitement de l'hypertension artérielle, caractérisé en ce que l'on met chacun des principes actifs, conjointement avec un véhicule physiologiquement acceptable et, le cas échéant, avec d'autres additifs ou adjuvants, sous une forme de présentation appropriée.